# EUROPEAN PATENT SPECIFICATION

(11) **EP 3 490 425 B1**
(45) Date of publication and mention of the grant of the patent: **20.05.2020**
(21) Application number: 17821736.0
(22) Date of filing: 31.10.2017
(51) Int. Cl.: A61B 1/00, A61B 1/012, A61B 1/05, A61B 1/06, A61B 1/07, G02B 23/24

(54) **FLEXIBLE ENDOSCOPE**
FLEXIBLES ENDOSKOP
ENDOSCOPE FLEXIBLE

(30) Priority: 01.11.2016 PL 41932816
(43) Date of publication of application: 05.06.2019
(73) Proprietor: EndoScope sp. z o.o., 87-100 Torun (PL)
(72) Inventor: MANKOWSKI, Szymon, 87-100 Torun (PL)
(74) Representative: Pawlowska, Justyna
(86) International application number: PCT/PL2017/000113
(87) International publication number: WO 2018/084730

(56) References cited:
- EP-A1- 2 604 175
- WO-A1-93/15648
- WO-A1-2016/182463
- US-A1- 2013 345 510

## Description

The invention relates to a flexible endoscope for specialized diagnostic and therapeutic procedures performed through hollow organs or body cavity.

There are known two types of medical endoscopes: inflexible endoscopes and flexible endoscopes such as bronchoscopes, gastroscopes and colonoscopes. Flexible endoscopes are equipped with a handle which, by means of a guiding connector, in most of cases conical shape, is connected to a insertion tube. The insertion tube has a form of flexible tube inside equipped with a bundle of functional channels, that are made inside and are performed through the tube, and it ends with a distal tip. Deflection of the distal tip is carried out by angulation wires which are pulled throughout the insertion tube and controlled by the knobs located on the handle. Functional channels are as follows: biopsy-suction channel, air channel, channel for flushing optical-illumination parts, or an water channel for flushing an observation area supplied from a separate pump. Optical fibers and electrical wires are also led through the endoscope to provide a light for video vision. Commonly functional channels, the optical fibers and the electrical wires come out from the handle and are connected to peripherals media by a bundle of terminals to provide the transmission of media for all functionalities, throughout the whole length of the endoscope until the end of the endoscope.

In known flexible endoscopes an elastic insertion tube ended with a tip, a handle, guiding connectors and the bundle of terminals stand as one inseparable part that means cannot be disassembled and removed. The known endoscopes have an optical-illumination elements which are not entirely separated from the functional channels, that means the elements cannot be removed from the endoscope. Due to the fact, the biopsy-suction channel often can be damaged during medical procedures that leads also to serious damages of the optical-illumination elements as well.

Endoscopy as a diagnostic tool, is unusually and commonly useful in diagnostics, but it involves a serious threat of infections mainly caused by mistakes made during cleaning, disinfection or sterilization of the devices. A particular design of endoscopes makes the procedure of cleaning and sterilizing very problematic. On the other hand, endoscopes are expensive so they are mostly produce as reusable devices. After medical procedure the endoscope is thoroughly cleaned, highly level disinfected and sterilized. This requires appropriate equipment as also technically and medically qualified staff. Critical parts and elements of the endoscopic devices that are susceptible for contamination and infection are these part with small diameters and significant length such as long functional channels of insertion tube. Because of complicated shapes and various materials that they are made of, specialized, more complicated methods of disinfection and sterilization are required.

Statistics show that practically every case of documented infection during endoscopy has been caused by abnormalities in cleaning, disinfection or sterilization. Many disinfectants that are used successfully for simple medical devices do not work well with more complex as endoscopes. Alcohols, aldehydes and high temperatures used in disinfection can induce a preservation of impurities and consequently formation of biofilms, especially on hard surfaces such as narrow functional channels. These disinfectants can be harmful for the personnel's health as well.

The increase of epidemiological risk associated with nosocomial infections require more effective methods against the transmission of infections. Unquestionably the most safe method here will be using disposable medical devices which after the medical treatment are disposed of.

Endoscopes that contain disposable elements are known. For example a flexible endoscope, where the distal end, of which in relation to the power supply unit, is connected separable with the working part of the insertion tube penetrating the interior of patient body, whereby separable connected are also pulled inside the handle and working part of the insertion tube optical fibers , water and air channels and angulation wires. In this solution optical fibers, channels, angulation wires have junctions where the working part of the insertion tube and the handle are connected.
This way the endoscope is equipped with detachable out of the handle that means removable and disposable insertion tube containing the bundles of functional channels, angulation wires, optical fibers. The handle and its connections require thorough cleaning, disinfection and sterilization before reuse. However, the problem of the high costs of components of disposable parts of the endoscope remains unresolved in the invention.

Patent application EP1765146 discloses a micro camera embedded in a sealed casing that is a reusable element for other medical devices, including an endoscope. This element includes a housing, lenses, semiconductor sensor, electronic controllers, internal electrical connectors. Internal and external electrical connectors include a number of pins or sockets arranged in pattern matching on the opposite surfaces so that the camera head can be connected and disconnected from the proper device. The micro camera is used in a reusable manner and the remaining components of the device in a disposable manner. The visual components in the endoscope, like micro cameras are separable from other endoscope parts including means for lighting, illumination function. Reusable visual elements are subjected to washing, disinfection and sterilization. According to the disclosed invention, electrical connectors of the micro controller may be exposed to body fluids and during disinfecting and sterilizing to hot steam or chemicals, which may require additional protection for micro cameras for endoscopic applications.

WO93/15648 discloses a flexible endoscope which is connected to an insertion tube in a form of an elastic tube ended with a distal tip and equipped with a bunch of functional channels.A flexible optical guide member, being positioned in the channel, is permanently or removable connected at a proximal end to the control module, and a disposable insertion tube removably attached at a proximal end to at least one of the control module and the optical guide member. The insertion tube includes a channel for receiving the optical guide member, the channel being closed at the distal end of the insertion tube. The optical guide member itself is related to the control module and it attached to the module at a proximal end of the endoscope. Each part has a similar diameter. The prevention of movement of the optical guide member is not provided and that is why the optical guide member is located in the endoscope unstable.

Hitherto, there is still a problem caused by high cost of using disposable specialized elements of endoscopes on the one hand and on the other hand there are necessary requirements of reducing the risk of contamination and infection in case of endoscopic elements used in a reusable manner.

The object of the invention is to provide the flexible endoscope which reduces such complications. Unexpectedly, applying a technically removable optical-illumination elements with specific construction in the endoscopes that allows to be separated from other means for other functions carried out through the functional channels of the insertion tube such as the water channel, air channel, suction channel, tools channel, enables to reduce the high cost of endoscopic procedures and the risk of contamination and infection. Invention enables to provide the flexible endoscope comprising a removable and independent optical-illumination module that is placed stable in a distal tip of the endoscope.

Thus according to invention, the flexible endoscope wherein the insertion tube is in a form of an elastic tube equipped with a bunch of functional channels ended with a distal tip, is characterized in that, the endoscope comprising a removable and independent optical-illumination module containing an elastic tube ended with a capsule while within, inside, the insertion tube an optical-illumination channel is formed. The insertion tube is divided into a distal tip, a bending section and a flexible part. Within the elastic tube there are performed optical fiber bundles and electrical wires ended with terminals for peripheral devices. A micro camera equipped with lenses and with terminals of optical fiber bundle and electrical wires is provided within the capsule. If the optical-illumination module is installed in the endoscope, the capsule is placed stable in a distal tip while the distal tip is formed by movable connected parts. The distal tip is formed by at least the two parts that are connected in separable or inseparable manner, wherein the parts, when connected, form the internal optical-illumination channel inside which the capsule of the optical-illumination module is installed. In a cross-section, a diameter of the capsule is larger than diameter of the elastic tube and furthermore the distal tip is equipped with a hook with length matched to narrowing being formed between the capsule and the elastic tube of the optical-illumination module. The features enables to provide stable position of the module with the camera relative to insertion tube what allows image recording from the stable positioned camera and also prevents from ejecting optical-illuminating module from insertion tube

Preferably, the elastic tube of the optical-illumination module is conducted inside and through the whole of the length of the insertion tube through the optical-illumination channel and in addition at least through a part of a distal guiding connector.

Preferably, the elastic tube of the optical-illumination module is conducted inside the insertion tube through the optical-illumination channel and in addition through the distal guiding connector and through a handle.

Preferably, on an outer surface of the capsule at least one fence is formed, preferably three fences, and through the whole length of the optical-illumination channel and on its inner contour at least one fence pocket is made, preferably three fence pockets, and the form of the fence pockets are matched to the fences of the capsule.

Preferably, the elastic tube of the optical-illumination module is coated with a metal sheath and/or a metal braid.

Preferably, in the capsule of the optical-illumination module a temperature recorder is placed.

Preferably, a mirror of variable adjustment of angle is attached to the distal tip.

Preferably, a bunch of functional channels of the insertion tube is formed as a multi-channel tube and is made as one solid element.

Preferably, in the distal guiding connector the outer input of optical-illumination module is made.

Preferably, a bunch of functional channels and an optical-illumination channel are formed by the inner walls of the handle.

Preferably, the functional channels of the insertion tube are particularly an air channel, a suction channel, a water channel for flushing lenses, a water channel for flushing an observation area, an angulation wires channel.

Preferably, a suction channel formed within the handle that sets continuation of a suction channel of the insertion tube functional channels is equipped with a filter.

According to the invention, the single use, disposable endoscope is provided with a sealed, independent and removable optical-illumination reusable module. This invention eliminates the main disadvantages of reusable endoscopes and at the same time it saves the most expensive components of the device such as micro-camera, optical fibers, electrical components and lenses which also stay reusable according to this invention.

The optical-illumination module, according to invention, can be apply to all known types of endoscopic procedures performed with flexible endoscopes by easily mounting it inside the device and thereby creating an universal, cost-effective and safe endoscopy system. In each of the embodiments of the invention, the optical-illumination module is positioned across the entire length of the insertion tube, independently of any functional channels provided in the insertion tube. In preferred embodiments of the invention, the module is continuously located further through the other elements of the endoscope such as the distal guiding connector, the handle, the proximal guiding connector. The output of the optical-illumination module is formed either in the element connecting the insertion tube and the handle, in the handle itself or in the proximal guiding connector.
The invention enables to substantially reduce the risk of infection transmission by facilitating and simplifying the daily usage of the endoscope particularly by the disposable design of the insertion tube containing functional channels, which are the most exposed to contamination and which constitute the greatest epidemiological risk in modern endoscopes. The preparation of the optical-illumination module for reuse in next medical procedure is much easier comparing to the preparation of entire endoscope according to current procedures as cleaning channels and performing tightness tests. Expensive endoscopic disinfectants are not required and specialized long-term storage cabinets for multiple-use endoscopes are not needed.

The invention can be used in diagnostics and also as a device for treatment procedures especially in gastrofiberoscopy, colonofiberoscopy, bronchofiberoscopy and if equipped with a variable angle mirror also in duodenoscopy. The invention make the endoscopic procedure more available and disseminates such important medical procedures.

The invention is shown in more details in the examples and drawings wherein:
Fig. 1 is an axonometric view of an endoscope, according to exemplary embodiment of the invention;
Fig. 2 is a 3D view of a distal end of a flexible part of an insertion tube with visible coating, according to exemplary embodiment of the invention;
Fig. 3 is a view of a bunch of functional channels, according to exemplary embodiment of the invention;
Fig. 4 is a half-section view of a distal guiding connector and a proximal end of an insertion tube, according to exemplary embodiment of the invention;
Fig. 5 is a view of a distal end of a flexible part of an insertion tube, a bending section and a distal end of an endoscope with is visible a distal end without metal mesh and polymeric coating, according to exemplary embodiment of the invention;
Fig. 6 is a longitudinal-section view of a snap-fit connection of a handle with distal guiding connector, according to exemplary embodiment of the invention;
Fig. 7 is a cross-section view of a handle with one valve visible, according to exemplary embodiment of the invention;
Fig. 8 is a cross-section view of the handle with visible filter,
Fig. 9 is w view of channels passing through a handle, a proximal end, and a bundle of connecting channels, according to exemplary embodiment of the invention;
Fig. 10 is an axonometric view of a part one of an distal tip, according to exemplary embodiment of the invention;
Fig. 11 is an axonometric view of a part two of an distal tip, according to exemplary embodiment of the invention;
Fig. 12 is a frontal view of the distal end of the endoscope with its second part deflected, according to exemplary embodiment of the invention;
Fig. 13 is a frontal view of the distal tip of the endoscope with its closed part one, according to exemplary embodiment of the invention;
Fig. 14 is a half-section view of a bellow of flexible part, according to exemplary embodiment of the invention;
Fig. 15 is a cross-section view of a flexible part of an insertion tube, according to exemplary embodiment of the invention;
Fig. 16 is a longitudinal-section view of a distal end of a distal tip of a flexible part of an insertion tube in a plane A shown in Fig. 2, according to exemplary embodiment of the invention;
Fig. 17 is a view of an insertion tube connection with a distal guiding connector, according to exemplary embodiment of the invention;
Fig. 18 is an axonometric view of an optical-illumination module, according to exemplary embodiment of the invention;
Fig. 19 is a view of a capsule of an optical-illumination module, according to exemplary embodiment of the invention;
Fig. 20 is a half-section of an endoscope tip with inserted end of an optical-illumination module, according to exemplary embodiment of the invention;
Fig. 21 is an axonometric view of an endoscope with an optical-illumination module inserted in a channel leading through a distal guiding connector, according to exemplary embodiment of the invention;
Fig. 22 is an axonometric view of an endoscope with a diagram of an optical-illumination module in a channel formed by all of an endoscope elements, according to exemplary embodiment of the invention;
Fig. 23 is a cross-section through a handle with visible one valve and an optical-illumination channel, according to exemplary embodiment of the invention;
Fig. 24 is a view of a proximal guiding connector and a connecting channels bundle with an optical-illumination module, according to exemplary embodiment of the invention;
Fig. 25 is an axonometric view of an optical-illumination module equipped with fences, according to exemplary embodiment of the invention;
Fig. 26 is a view of a Bunch of functional channels with fence pockets of an optical-illumination module, according to exemplary embodiment of the invention;
Fig. 27 is an axonometric view of a part one of an endoscope
with fence pockets of an optical-illumination channel, according to exemplary embodiment of the invention;
Fig. 28 is an axonometric view of a part two of an endoscope provided with a hook, according to exemplary embodiment of the invention;
Fig. 29 is a half-section view of an distal tip with a capsule of an optical-illumination module inserted in and stabilized by a hook, according to exemplary embodiment of the invention;
Fig. 30 is a half-section of an distal tip equipped with an adjustable angle mirror, according to exemplary embodiment of the invention.

### Example 1

### a) flexible endoscope structure.

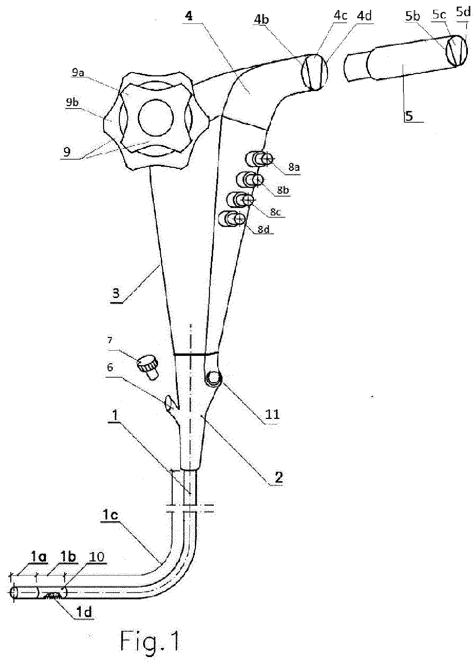

As presented in the Figure 1, the flexible endoscope comprises an insertion tube 1 in a form of elastic tube, a guiding connection in form of a distal guiding connector 2, a handle 3, a proximal guiding connector 4, a connecting channels bundle 5 having a form of a bundle of elastic tubes which make the outlet of the endoscope function channels that connect peripheral devices such as suction device and pumping water and air device. On the handle 3 are placed valves 8 and knobs which control angulation wires 9.

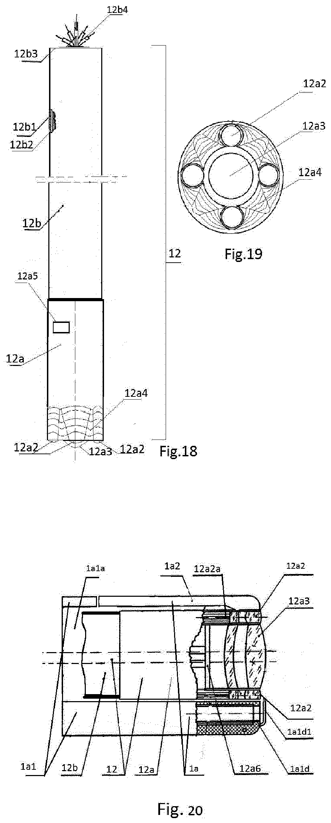

As presented in the Fig. 18, the endoscope is equipped with a removable and an independent optical-illumination module 12, which is performed in a separated optical-illumination channel. The optical-illumination module 12 includes an elastic tube 12b, ended with a capsule 12a. The optical-illumination module 12 is described in this example further.

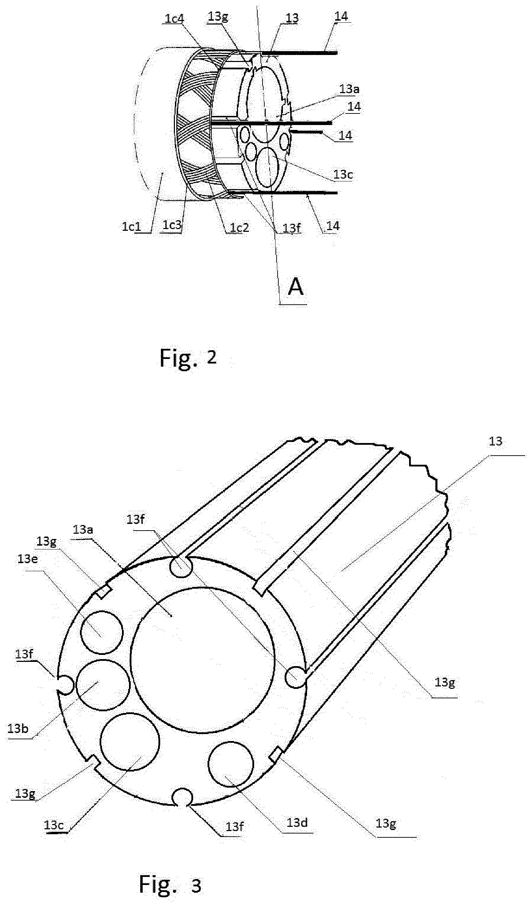

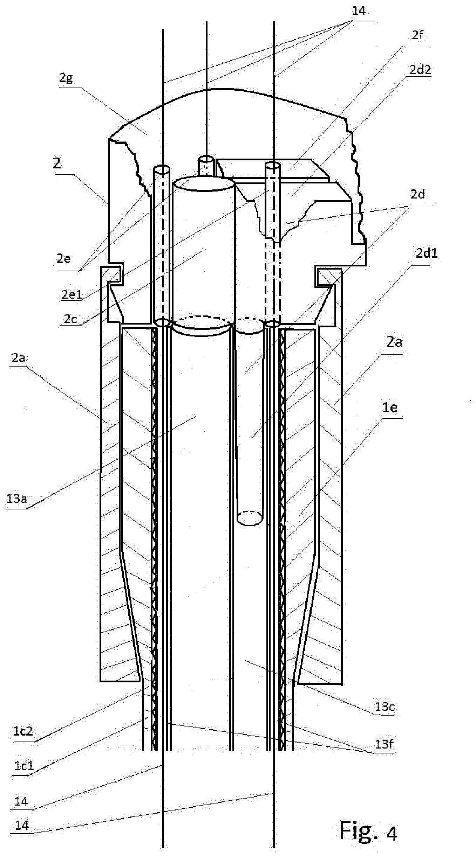

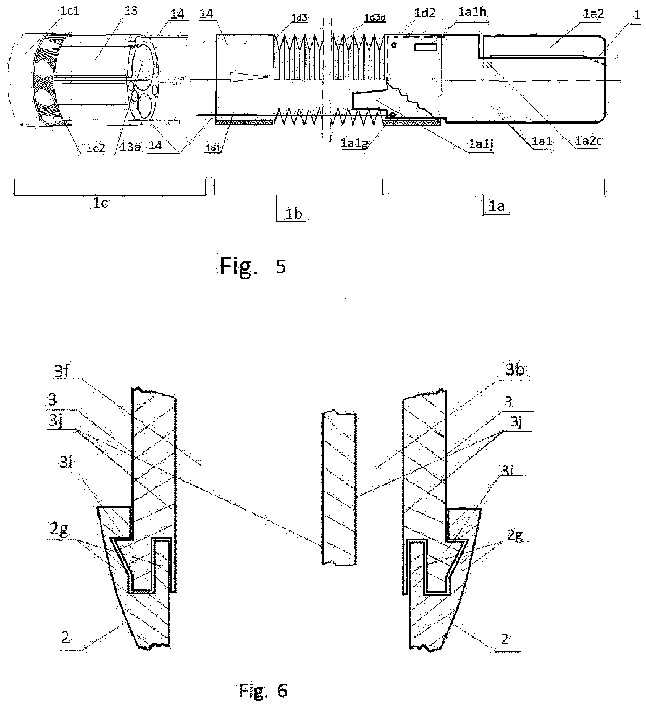

The insertion tube 1 is divided into a distal tip 1a, a bending section 1b, a flexible part 1c. As it is shown in the Fig. 2, bunch of functional channels 13 are placed inside the insertion tube 1. The bunch of functional channels 13 is formed as a multichannel tube made of flexible polymer, can be low density polyethylene, which is made as one solid element Fig. 3. Bunch of function channels 13 of insertion tube 1 includes: air channel 13b, suction channel 13c, water channel for flushing lenses 13d, water channel for flushing an observation area 13e, angulation wires channels 13f and pockets 13g for flexible portion of bellow 1d3. Insertion tube 1 is provided with an independent optical-illumination channel 13a. Bunch of functional channels 13 is conducted through the insertion tube 1 connecting channels in the handle 3 by the distal guiding connector 2, and from the other side connecting channels in the distal tip 1a, as shown in the Fig. 4 and Fig. 5.

The function of the distal guiding connector 2 is to attach the insertion tube 1 to the handle 3 and to connect the functional channels: suction, air, for lens flushing, for flushing an observation area and also angulation wires channels between the insertion tube 1 and the handle 3.

The optical-illumination channel 13a and the angulation wires channels 13f of the insertion tube 1 are respectively connected to the optical-illumination channel 2c and the angulation wires channels 2e provided in the distal guiding connector 2 without having necessary sealing of the joint with additional elements which could cause an effect of narrowing in the cross-section of the channels. The remaining functional channels 13 of the insertion tube 1: suction channel 13c, air 13b, water channels for flushing lenses 13d and water channel for flushing an observation area 13e are connected to respective channels of the distal guiding connector 2 by means of sealed distal joints 2d1 which are formed as fittings shaped parts adjusted to their internal diameters, what is presented in the Fig. 4.

In the distal guiding connector 2 the change of direction or size of all the functional channels of the insertion tube 1 is performed to adjust them to the construction and a size of the handle 3 ,as shown in Fig. 4 for the suction channel 2d and its proximal joint 2d2 and the distal joint 2d1. The channel for angulation wire 2e1 in the distal guiding connector 2 is led under the suction channel 2d. The distal guiding connector 2 is provided with an entrance of a biopsy channel 6 which is connected with the suction channel and is used for inserting endoscopic instruments through it. In the distal guiding connector 2 is made an entrance 11 for the optical-illumination module, as shown in the Fig.1.

As presented in the Fig. 6, the proximal flange 2g of the distal guiding connector 2 containing around the whole perimeter the outer wall of the handle 3 is connected inseparably with the distal end of the handle 3 by snap-fits 3i.

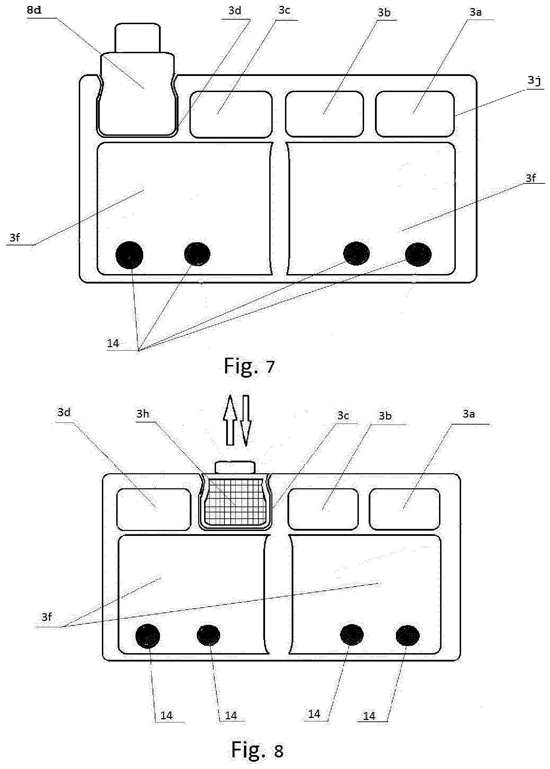

As shown in the Fig. 7, in the handle 3 functional channels are provided extending the bunch of functional channels 13 of the insertion tube 1 which are: air channel 3d, suction channel 3c, water channel for flushing lenses 3b, water channel flushing an observation area 3a, angulation wires channels 3f. The function channels of the handle 3 are formed by the inner walls of the handle 3j and form an integral part with it.

Functional channels of the handle 3 are provided with channel valves 8 (it can be piston valves) such as an air channel valve 8d, suction channel valve 8c, a water channel valve for flushing lenses 8b, a water channel valve for flushing an observation area 8a. The flow of media in the corresponding channel is closed or opened by channel valves 8.

As presented in the Fig. 8, the suction channel 3c in the handle 3 is additionally provided with a removable filter 3h distally mounted from the suction channel valve 8c. It is a "trap" preventing clogging of the suction channel valve 8c during operating the endoscope and also is it a place to retain endothelial tissue fragments such as polyps when the suction function is in use.

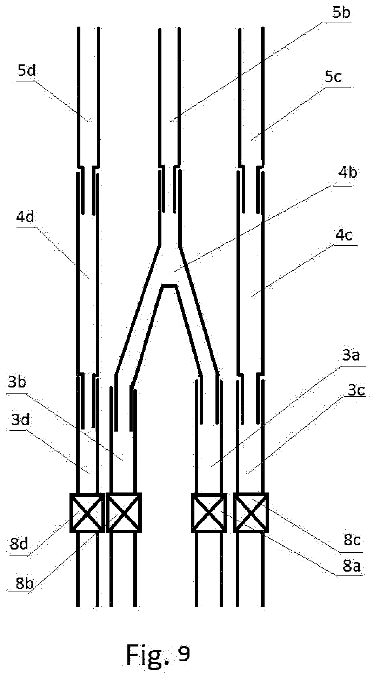

The handle 3 is connected inseparably to the proximal guiding connector 4 and the functional channels 3a, 3b, 3c, 3d of the handle 3 connecting to the aqueous channels 5b, suction 5c and air 5d of the connecting channels bundle 5 through the functional channels of the proximal guiding connector 4: air channel junction 4d, water channel junction 4b, suction channel junction 4c. In the proximal guiding connector 4, the water channel 4b is separated and connected to the water channel for flushing lenses 3b and the water channel for flushing an observation area 3a of the handle 3, as shown in the Fig. 9.

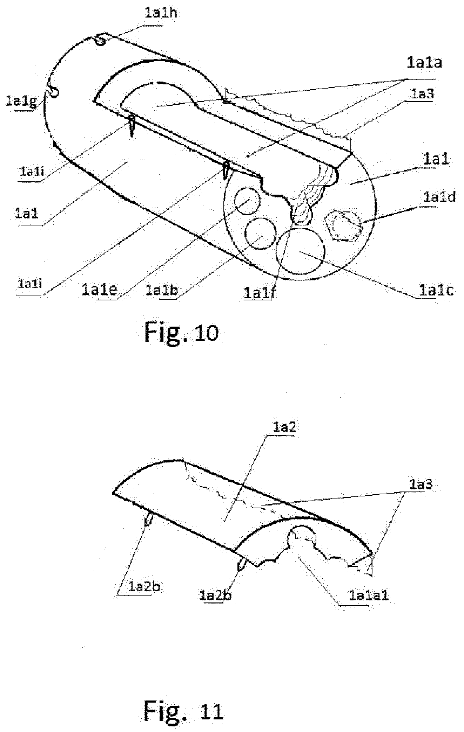

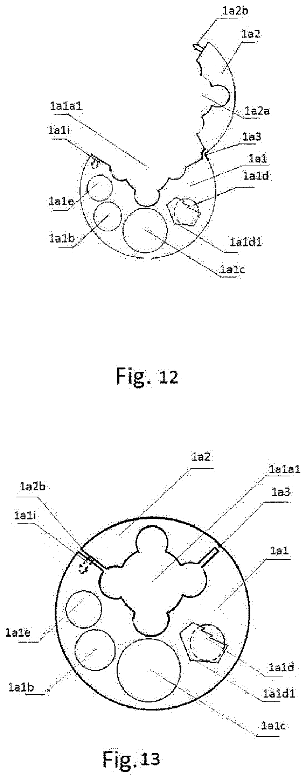

Fig. 10 and Fig. 11 show the distal tip 1a of the endoscope consisting two parts - a first part 1a1 and a second part 1a2, which are connected to one of their edges by a connecting element 1a3 formed on them by a distal tip 1a narrowing between a part one 1a1 and a part two 1a2 and being a flexible polymer band permanently assembled with both parts of the distal tip 1a1 and 1a2. In this way, the connecting element 1a3 is formed, through which both parts 1a1 and 1a2 can be tilted as shown in Fig. 12. As shown in the Fig. 10, Fig.1 1, Fig.12 and Fig. 13, opposite to the connecting member 1a3 the first part 1a1 is provided with pins for the pin sockets 1a1i, the second part 1a2 with pins 1a2b, which guarantee for good connection of the two parts by means of a separable connection. As shown in the Fig. 10, an optical-illumination channel 1a1a is provided for inserting the capsule 12a of the optical-illumination module 12 within the distal tip 1a. This channel is formed after closure of both part one 1a1 and part two 1a2 of the distal tip 1a.

The precise alignment of the outer surface of the capsule 12a to the inner surface of the distal tip 1a provide a stable positioning of the capsule 12a in the distal tip 1a. In this way, both parts of the endoscope are connected after sealing the first part 1a1 and the second end 1a2 of the distal tip 1a. In addition, the distal tip 1a is provided with angulation wire sockets 1a1g for fixing four of angulation wires causing bending in perpendicular, horizontal and vertical planes.

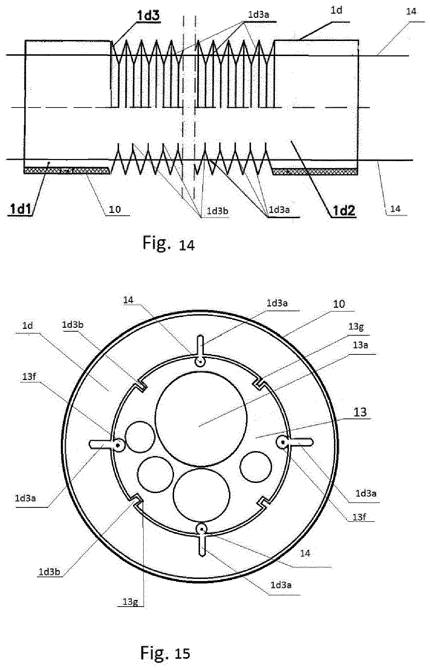

As shown in the Fig. 14 and Fig. 15, a bending section 1b of the insertion tube comprises a bellow of flexible part 1d in the form of a bellow which is shielded by a protector 10 made from a flexible polymer. In the bending section 1b, further ends of the bunch of functional channels 13 of the insertion tube 1 and further sections of the angulation wires 14 are led. The bellow of flexible part 1d, by its properties, improves and stabilizes the bending of the bending section 1b of the insertion tube. As shown in the Fig. 15, the bellow of flexible part 1d in the form of a bellow has angulation wire pockets ld3a for the angulation wires 14 which are placed in series in the longitudinal axis of the bellow of flexible part 1d against the channels of the respective angulation wires channels 13f. The appropriate position of the bellow of flexible part 1d is provided by bellow ribs 1d3b which are located in pockets 13g created around the walls forming the contour of the bunch of functional channels 13 of the insertion tube 1. In this solution of the bending section 1b of the insertion tube 1 the pulling of wires through the holes in the bending section 1b is not required, because pushing a bellow of flexible part 1d on the bunch of functional channels 13 can be sufficient.

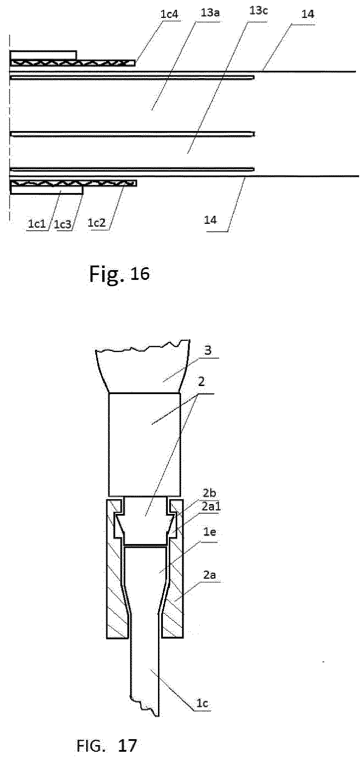

As shown in the Fig. 2 and Fig. 16, the flexible part 1c of the insertion tube 1 is covered with a metal mesh 1c2 and a polymeric coating 1c1 made of polyethylene. At the distal end of the flexible part 1c of the insertion tube, the polymeric coating 1c1 is ended with the boundary 1c3 exposing the metal mesh 1c2 on which the proximal end 1d1 of bellow 1d is put. The distal end 1d2 of the bellow of flexible part 1d is connected to the distal tip 1a of the endoscope whilst the bunch of functional channels 13 with angulation wires 14, without polymeric coating 1c1 and the metal mesh 1c2 passes inside the bellow of flexible part 1d and is connected to the channels of the distal tip 1a of the endoscope by means of appropriate suction channel junction 1a1j Fig. 5.

The flexible part 1c of the insertion tube 1 at the proximal end having a circular widening formed by the polymer coating 1c1 and having a form of a flange 1e forming together with a bushing 2a a connection of the insertion tube 1 with the bellow 2 of the flexible part 1c. The bushing 2a is equipped with a snap-fit socket 2a1 securing a snap-fit 2b of the distal guiding connector 2 and its inner shape is adjusted to the flange shape 1e Fig. 17. The shape of the flange 1e in a cross-section is different than a spherical shape, here for example quadrilateral preventing the rotation of the insertion tube 1 in relation to the distal guiding connector 2 and the handle 3.

As shown in the Fig. 18 and Fig. 19, the optical-illumination module 12 comprises a capsule 12a and the elastic tube 12b. As shown in Fig. 20, the capsule 12a comprises a micro camera 12a6 with a lenses 12a3, distal segments of the optical fibers 12a2a with lenses 12a2 for illumination the observed field. The temperature recorder 12a5 is located in the capsule 12a. The temperature recorder 12a5 registers temperature increasing as a function of time, which allows for assessment of sterilization level or disinfection process. The distal end of the capsule 12a is formed by the oblique planes 12a4 to prevent protrusion beyond the frontal plane of the distal tip 1a and rotating as well and represent a contour of the inner shape of the oblique planes 1a1f of the distal tip 1a. The oblique planes 12a4 are located between the lenses of optical fibers 12a2.

Through the elastic tube 12b of the optical-illumination module 12 optical fiber bundles 12b4 and electrical wires 12b3 are led and connected by the suitable terminals to the peripheral devices. The elastic tube 12b is covered with a polymer coating 12b1 made of polytetrafluoroethylene PTFE or a thermoplastic elastomer covering a metal braid 12b2. The electrical wires 12b3 and the optical fiber bundles 12b4 are protected by the polymer coating 12b1 and the braid 12b2. Using a braid of various densities decreasing towards to the capsule 12a allows the elastic tube 12b to be gradually flexile so that its flexibility is increased toward the capsule 12a.

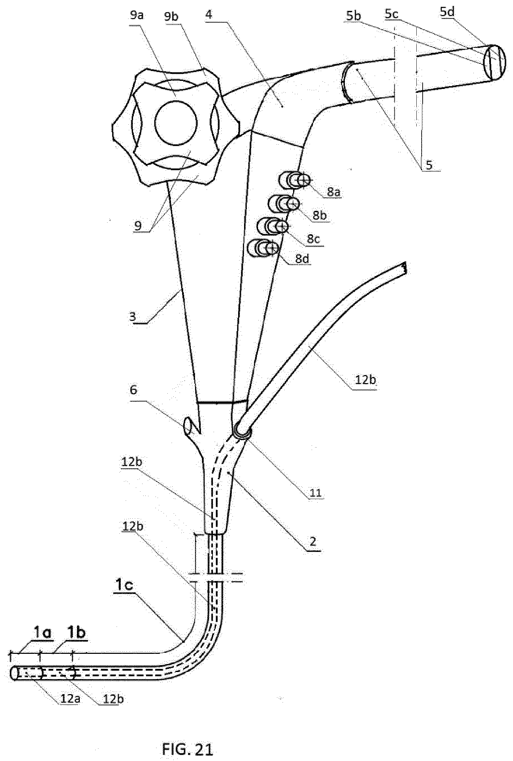

The optical-illumination module 12 is led through the distal guiding connector 2 in such a fashion that the capsule 12a and the elastic tube 12b of the optical-illumination module 12 are inserted through the input of the optical-illumination module 11, an opening for the optical-illumination module 12 formed in the wall of the distal guiding connector 2, which leads to its optical-illumination channel 2c and further down the optical-illumination channel 13a of the insertion tube 1 to the distal tip 1a, as it is shown in Fig. 21.

The optical-illumination module 12 is connected via optical and electrical connectors to peripherals such as a computer, image processor, light source. A light from the light generating device is transmitted through the optical fiber bundles 12b4 of the optical-illumination module 12 to the end of the endoscope. The optical fiber bundles 12b4 is connected to light source by means of an optical connector. The signal from micro camera 12a6 is transmitted by electrical wires 12b3 of the optical-illumination module 12 via electrical connectors detachably connected to a peripheral device which may be a computer or dedicated image processor only. The micro camera 12a6 and PC can be connected via USB or S-video bus.

### b) Mounting and unmounting the optical-illumination module.

As presented in the Fig. 21, the optical-illumination module 12 is inserted into the optical-illumination channel 2c through the input 11 through the distal guiding connector 2. Further the optical-illumination module 12 is pulled in the optical-illumination channel 13a of the insertion tube 1 until the capsule 12a is inserted into the optical-illumination channel 1a1a in a distal tip 1a. Before closing parts 1a1 and 1a2 of the distal tip 1a the positioning correction should be made in order to achieve a good quality of video registration. The optical-illumination module 12 is mounted in such a way that after being installed in the endoscope, the capsule is placed stable in a distal tip while the distal tip is formed by movable connected parts. Parts 1a1 and 1a2 are connected with pins 1a2b and pin sockets 1a1i. After closing of the part one 1a1 and the part two 1a2 of the distal tip 1a a stable connection is created by the precise alignment of the outer surface of the capsule 12a to the inner surface of the distal tip 1a. The oblique planes 1a1f of the distal tip adjusted to the oblique planes 12a4 of the capsule 12a prevent from changing position and protruding the capsule beyond the forehead of the distal tip 1a.

After medical procedure the optical-illumination module 12 is unmounted. The distal tip 1a is opened so that the part one 1a1 is disengaged from the part two 1a2 by pulling off the pins 1a2b from the pins sockets 1a1i, thus allowing the part one 1a1 and part two 1a2 of the distal tip 1a to be folded back and for disengaged the capsule 12a from the distal tip 1a. The optical-illumination module 12 is then pulled out back through the input of optical-illumination module 11 of the distal end 2, preciously having disconnected the terminals from the peripheral devices.

The optical-illumination module 12 when separated should be disinfected or sterilized while the remaining parts of the endoscope are disposable so can be disposed of. The endoscope constructed accordingly with this invention guarantee safe medical procedures for the patients and the medical staff, also reduction of costs of using expensive elements.

### Example 2

### a) Flexible endoscope structure.

The endoscope is constructed as described in the Example 1 with exception that the optical-illumination module 12 is pulled in throughout the entire length of the endoscope.

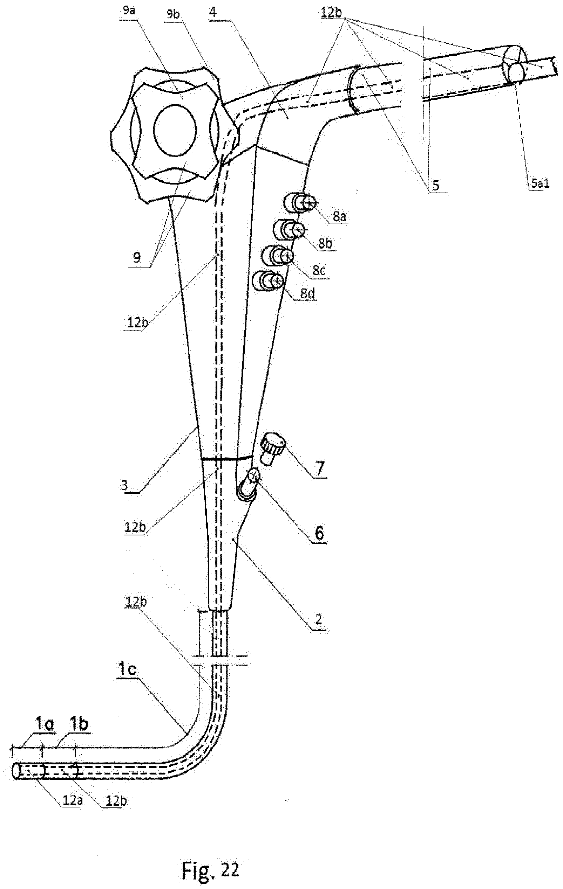

As shown in the Fig.22, the optical-illumination module 12 is located in the optical-illumination channel formed in the following parts of the endoscope: in insertion tube 1, distal end 2, handle 3, proximal guiding connector 4, connecting channels bundle 5.

In the distal end 2 an optical-illumination channel 2c is formed and connected tangentially with the optical-illumination channel 13a of the insertion tube 1 and with optical-illumination channel of the handle 3e.

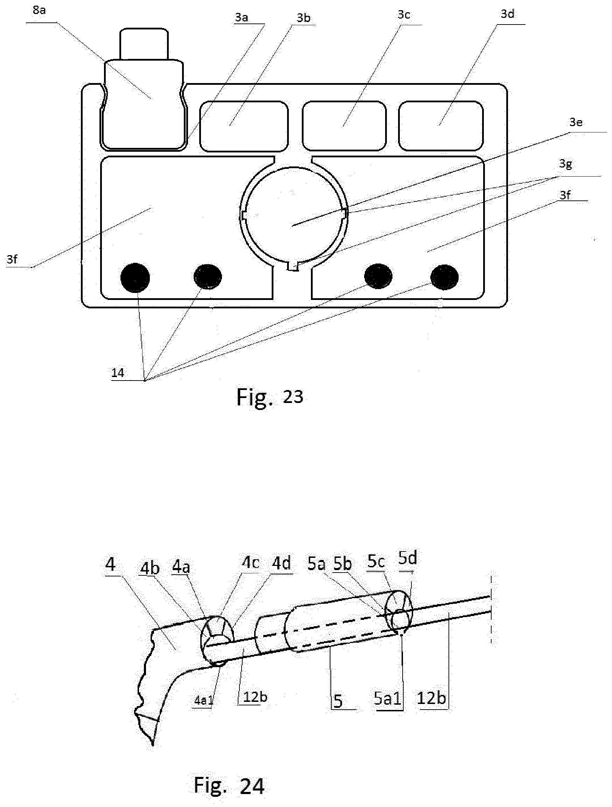

As shown in the Fig. 23, functional channels: air channel 3d, suction channel 3c, water channel for flushing lenses 3b, water channel for flushing an observation area 3a, angulation wire channels 3f, and optical-illumination module channel 3e are formed. The walls of all channels are created by inner walls of the handle 3. The handle 3 is equipped with valves 8 for the following channels: air channel valve 8d, suction channel valve 8c, water channel valve for flushing lenses 8b, water channel valve for flushing an observation area 8a.

Functional channels of the handle 3 are connected through the proximal end 4 with the channels of the connecting channel bundle 5 through the water channel junction 4b, the suction channel junction 4c, the air channel junction 4d. On the other hand, the optical-illumination channel 4a of the proximal guiding connector 4 has its outlet 4a1. The bundle of the connecting channels 5 is provided with a longitudinal slot 5a1 of the optical-illumination channel 5a through the long axis, as presented in the Fig. 24.

### b) Mounting and unmounting of the optical-illumination module.

As shown in the Fig. 22 and Fig. 24, the optical-illumination module 12 is inserted through the optical-illumination channel outlet 4a1 into the optical-illumination channel 4a of the proximal guiding connector 4. The optical-illumination module 12 is led in optical-illumination channel through following elements of the endoscope as: optical-illumination module channel 3e of the handle 3, optical-illumination channel 2c of the distal guiding connector 2, the optical-illumination channel 13a which is led in the insertion tube 1 up to the distal tip 1a. Upon insertion of the optical-illumination module 12 into the endoscope, the capsule 12a is detachably mounted to the distal tip 1a. After proper adjustment of the capsule 12a, before the closing of the two parts 1a1, 1a2 these are connected using the pins 1a2b and the corresponding pins sockets 1a1i.

It is possible to use another detachable or inseparable connection to connect the part one 1a1 to the part two 1a2 of the distal tip 1a. The inseparable connection can be realized by using pins 1a2b instead of hooks 1a2c. Application of an inseparable connection imposes that before open the part one 1a1 and part two 1a2 of the distal tip 1a destroying of connecting element 1a3 after medical procedure by cutting it. This will prevent reuse of the endoscope and only re-use of the unmounted optical-illumination module, which minimizes the risk of contamination and infection through endoscopic components difficult to clean, which are in this solution designed to be used once. Precise adjustment of the outer surface of the capsule 12a to the inner surface of the distal tip 1a makes a stable connection between them which is created after closing part one 1a1 and part two 1a2 of the distal tip 1a. The oblique planes 1a1f adjusted to the oblique planes 12a4 the capsule 12a prevent from protrusion of the capsule beyond the forehead of the distal tip 1a.

As shown in Fig. 24, after the capsule 12a has been attached to the distal tip 1a, the elastic tube 12b is detachably mounted by pushing to the slot 5a1 into the optical-illumination channel 5a of the connecting channels bundle 5 and thus both elements have connection to the peripheral devices. After medical procedure the optical-illumination module 12 is unmounted and cleaned, disinfected or sterilized. Parts 1a1, 1a2 of the distal tip 1a are disengaged by pulling off the pins 1a2b from the pins sockets 1a1i, which allows the parts 1a1, 1a2 of the distal tip 1a to be deflected and the capsule 12a disconnected from the distal tip 1a or by cutting the connecting element 1a3 and in the case of an inseparable connection - hooks. The optical-illumination module 12 is then extended from optical-illumination channel through the slot 5a1 of the connecting channel bundle 5 and pulled out of the proximal guiding connector 4 through the opening of the optical-illumination channel outlet 4a1, after disconnecting the terminals from the peripheral devices.

### Example 3

### a) flexible endoscope structure.

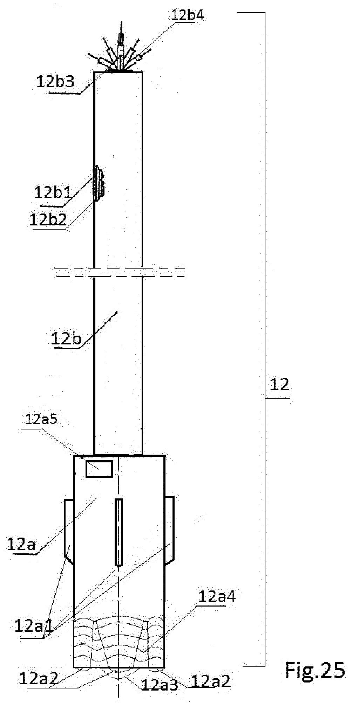

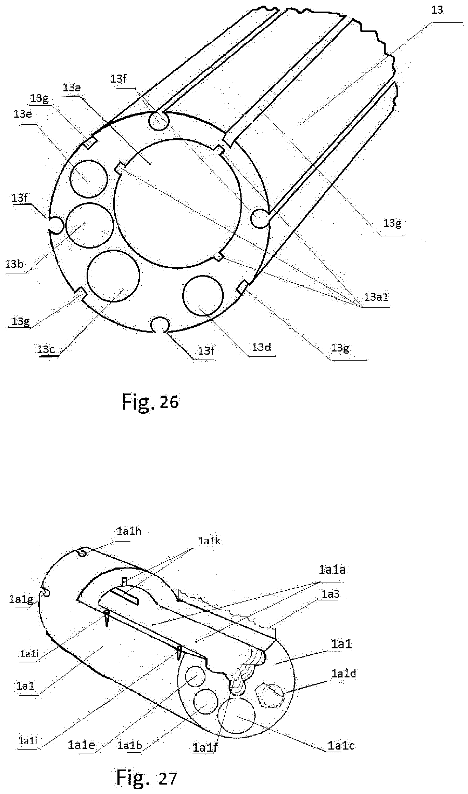

The endoscope is constructed as described in Example 1 except that three fences 12a1 are formed on the outer surface of the capsule 12a and over the entire length of the insertion tube 1 an optical-illumination channel 13a is created, on which outer contour the three fence pockets 13a1 are formed matching to fences 12a1 of the capsule 12a Fig. 25 and Fig. 26. In the distal tip 1a on the outline of the optical-illumination channel 1a1a are also three fence pockets 1a1k matched to the fences 12a1 of the capsule 12a, as shown in the Fig. 27.

The fences 12a1 facilitate the insertion of the optical-illumination module through the optical-illumination channel in a suitable position relative to the longitudinal axis of the insertion tube 1. Thus, the capsule 12a is inserted into the optical-illumination channel 1a1a of the distal tip 1a in a strictly specified position for precise adjusting the oblique surfaces of the capsule 12a4 to the oblique surfaces of the insertion tube 1a1f. Placing the optical-illumination module 12 only in a single, repeatable position allows for proper placement of the guides with the correct track selections, which ensures correct recording of the image during endoscopic surgery.

It is also possible to use fences in an endoscope constructed as described in Example 2 except that appropriate pockets are adjusted to the size and shape of the fences and are formed in the outline of the optical-illumination channel formed by the proximal guiding connector 4, the handle 3, the distal guiding connector 2, distal tip 1a, bending section 1b, flexible part 1c.

### b) Mounting and unmounting the optical-illumination module.

As shown in Fig. 20, the optical-illumination module 12, after being inserted through the input of the optical-illumination module 11 into the distal guiding connector 2, is led through the optical-illumination channel 13a of the insertion tube 1 so that the capsule 12a is inserted into the optical-illumination channel 1a1a of the distal tip 1a. The fences 12a1 improves the insertion of the optical-illumination module 12 so that it is not necessary to correct the positioning of the capsule 12a prior to closing both parts 1a1 and 1a2 of the distal tip 1a. Parts 1a1 and 1a2 of the distal tip 1a are connected by pins 1a2b and corresponding slots 1a1i. After the medical procedure, the optical module 12 is removed. Parts 1a1 na 1a2 of the distal tip 1a are disengaged by pulling of the pins 1a2b from the pins sockets 1a1i, which allows the part one 1a1 and part two 1a2 of the distal tip 1a to be tilted and the capsule 12a to be detached from the distal tip 1a. The optical-illumination module 12 is then pulled of backwards through the input of the optical-illumination module 11 of the distal end 2 previously having disconnected terminals from the peripheral devices.

### Example 4

### a) flexible endoscope structure.

The endoscope is constructed as described in the Example 1 except that the diameter of the capsule 12a in cross-section is greater than the diameter of the elastic tube 12b, and the distal tip 1a is provided with a hook 1a2c of which the length is adapted to the narrowing in the capsule 12a with the elastic tube 12b of the optical-illumination module 12, what is presented in the Fig. 25, 28, 29.

### b) Mounting and unmounting the optical-illumination module.

The optical illumination module 12 inserted into the endoscope and removed as described in Example 1 except that after inserting the capsule 12a into the optical-illumination channel 1a1a of the distal tip 1a and closing the parts 1a1 and 1a2, the hook 1a2c of the part two 1a2 of the distal tip 1a prevents the capsule 12a from moving back by supporting its rear wall which additionally stabilizes the capsule 12a in the endoscope.

### Example 5

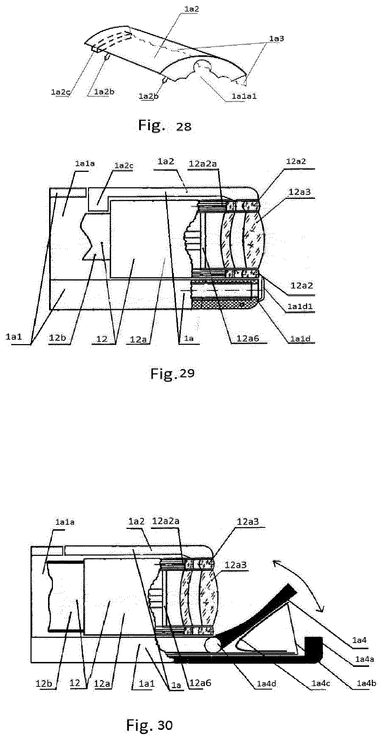

The endoscope is constructed as described in the Example 1 or 2 except that a variable angle mirror 1a4 is mounted to the distal tip 1a, what is presented in the Fig. 30. Because of that the conversion of the straightforward observation used in the optical-illumination module to side observation is possible. Tilting the mirror at a predetermined angle is possible by the spring 1a4c formed from a resilient metal flat bar, interacting with the angulation wire 1a4b. The angulation wire is driven by a dedicated channel through the insertion tube 1 and the distal end 2 to the handle 3. The pulling of the angulation wire 1a4b causes the horizontal arrangement of the mirror 1a4 and covering it with the cover 1a4a, which allows for straightforward observation. Smooth, cylindrical cover protects the patient from injury, facilitates the insertion of the endoscope and protects the mirror from damage. At the operator's choice, when the angulation wire 1a4b is released, the spring 1a4c of the mirror 1a4 sets up this mirror in predetermined angle and provides the ability to observe in side direction. This solution allows to be used in duodenoscopy and medical treatment on bile ducts, as well as observation and treatment in difficult-to-reach areas, such as the folds of the large intestine. It also allows to see straightforward or to the side during the same test or treatment. The optical-illumination module 12 is mounted and unmounted as described in Example 1 or 2.

### References to the drawing

- 1: Insertion tube
1a Distal tip
1a1 Part one
1a1a Optical-illumination channel
1a1a1 Optical-illumination channel outlet
1a1b Air channel outlet
1a1c Suction channel outlet
1a1d Water channel outlet for flushing lenses
1a1d1 Nozzle
1a1e Water channel outlet for flushing an observation area
1a1f Oblique planes
1a1g Angulation wire socket
1a1i Pin socket
1a1j Suction channel junction
1a1k Fence pockets
1a2 Part two
1a2a Optical-illumination channel outlet
1a2b Pin
1a2c Hook
1a3 Connecting element
1a4 Mirror
1a4a Cover
1a4b Angulation wire
1a4c Spring
1a4d Joint of at least one degree of freedom
1b Bending section
1c Flexible part
1c1 Polymeric coating
1c2 Metal mesh
1c3 Polymeric coating boundary
1c4 Metal mesh boundary
1d Bellow of flexible part
1d1 Proximal end
1d2 Distal end
1d3 Flexible portion of bellow
1d3a Angulation wires pockets
1d3b Bellow ribs
1d3c Angulation wires channels
1e Flange
- 2: Distal guiding connector
2a Bushing
2a1 Snap-fit socket
2b Snap-fit
2c An optical-illumination channel
2d Suction channel
2d1 Distal joint
2d2 Proximal joint
2e Angulation wires channels
2e1 Channel for angulation wire passing throughout suction channel
2f Proximal joint of channel for flushing lenses
2g Proximal flange
- 3: Handle
3a Water channel for flushing an observation area
3b Water channel for flushing lenses
3c Suction channel
3d Air channel
3e Optical-illumination module channel
3e1 Slot
3f Angulation wires channel
3g Fence pockets
3h Filter
3i Snap-fit
3j Inner wall of the handle
- 4.: Proximal guiding connector
4a Optical-illumination channel
4a1 Optical-illumination channel outlet
4b Water channel junction
4c Suction channel junction
4d Air channel junction
- 5: Connecting channels bundle
5a Optical-illumination channel
5a1 Slot
5b Water channel
5c Suction channel
5d Air channel
- 6: Entrance of the biopsy channel
- 7: Plug
- 8: Channels valves
8a Water channel valve for flushing an observation area
8b Water channel valve for flushing lenses
8c Suction channel valve
8d Air channel valve
- 9: Knobs for angulation wires control
9a Knob for control the angulation wires in horizontal
9b Knob for control the angulation wires in vertical
- 10: Protector
- 11: Input of optical-illumination module
- 12: Optical-illumination module
12a Capsule
12a1 Fences
12a2 Lenses of optical fibers
12a2a Optical fiber
12a3 Lenses
12a4 Oblique planes
12a5 Temperature recorder
12a6 Micro camera
12b Elastic tube
12b1 Polymer coating
12b2 Braid
12b3 Electrical wires
12b4 Optical fiber bundles
- 13: Bunch of functional channels
13a Optical-illumination channel
13a1 Fence pockets
13b Air channel
13c Suction channel
13d Water channel for flushing lenses
13e Water channel for flushing an observation area
13f Angulation wires channels
13g Pockets
- 14: Angulation wire

## Claims

1. A flexible endoscope comprising a handle, which is connected to an insertion tube by a distal guiding connector, wherein the insertion tube is in the form of an elastic tubular member equipped with a bunch of functional channels and ended with a distal tip, the endoscope further comprising a removable and independent optical-illumination module (12) containing an elastic tube (12b) ended with a capsule (12a) and the insertion tube (1) is divided into a distal tip (1a), a bending section (1b) and a flexible part (1c) while within the insertion tube (1) an optical-illumination channel (13a) is formed, and in addition within the elastic tube (12b) an optical fiber bundles (12b4) and electrical wires (12b3) are provided and ended with terminals for peripheral devices, wherein a micro camera (12a6) equipped with lenses (12a3) and with terminals of optical fiber bundle (12b4) and electrical wires (12b3) is provided within the capsule (12a), wherein in a cross-section a diameter of the capsule (12a) is larger than the diameter of the elastic tube (12b) and moreover if the optical-illumination module (12) is installed in the endoscope the capsule (12a) is placed stable in a distal tip (1a) that is formed by movable connected parts, wherein the distal tip (1a) is formed by at least the two parts that are connected in separable or inseparable manner and these parts, when connected, form the internal optical-illumination channel (1a) inside which the capsule (12a) of the optical-illumination module (12) is installed and furthermore the distal tip (1a) is equipped with a hook (1a2c) having a length matched to a narrowing formed between the capsule (12a) and the elastic tube (12b) of the optical-illumination module (12) to prevent the capsule from moving back by supporting its rear wall.

2. The endoscope according to the claim 1, wherein the elastic tube (12b) of the optical-illumination module (12) is conducted inside and through the whole of the length of the insertion tube (1) through the optical-illumination channel (13a), and at least through a part of a distal guiding connector (2).

3. The endoscope according to the claim 1, wherein the elastic tube (12b) of the optical-illumination module (12) is conducted inside the insertion tube (1) through the optical-illumination channel (13a) and through the distal guiding connector (2) and through a handle (3).

4. The endoscope according to any of the claims 1-3, wherein on an outer surface of the capsule (12a) at least one fence (12a1) is formed, preferably three fences, and through the whole length of the optical-illumination channel (13a) and on its inner contour at least one fence pocket (13a1) is made, preferably three fence pockets and the form of the fence pockets (13a1) are matched to the fences (12a1) of the capsule (12a).

5. The endoscope according to any of the claims 1-4, wherein the elastic tube (12b) of the optical-illumination module (12) is coated with a metal sheath and/or a metal braid.

6. The endoscope according to any of the claims 1-5, wherein in the capsule (12a) of the optical-illumination module (12) a temperature recorder (12a5) is placed.

7. The endoscope according to any of the claims 1-6, wherein a mirror (1a4) of variable adjustment of angle is attached to the distal tip (1a).

8. The endoscope according to the claims 1-7, wherein a bunch of functional channels (13) of the insertion tube (1) is formed as a multi-channel tube and is made as one solid element.

9. The endoscope according to the claim 2, wherein in the distal guiding connector (2) the outer input of optical-illumination module (11) is made.

10. The endoscope according to the claim 3, wherein a bunch of functional channels (3a, 3b, 3c, 3d) and an optical-illumination channel (3e) are formed by the inner walls of the handle (3).

11. The endoscope according to the claim 8, wherein the functional channels (13)of the insertion tube (1) are particularly an air channel (13b), a suction channel (13c), a water channel for flushing lenses (13d), a water channel for flushing an observation area (13e), an angulation wires channel (13f).

12. The endoscope according to the claim 11, wherein a suction channel (3c) formed within the handle (3) that sets continuation of a suction channel (13c) of the insertion tube (1) functional channels (13) is equipped with a filter (3h).

## Patentansprüche

1. Ein flexibles Endoskop mit einem Handgriff, der über einen Führungsverbinder mit einer Sonde in Form eines flexiblen Kabels mit einem Bündel von Funktionskanälen verbunden ist, das mit einem distalen Endstück abschließt, **dadurch gekennzeichnet, dass** es mit einem abnehmbaren und in sich geschlossenen Optik- und Beleuchtungsmodul (12) ausgestattet ist, das ein flexibles Kabel (12b) enthält, das mit einer Kapsel (12a) endet, und die Sonde (1) in ein distales Ende (1a), ein Biegesegment (1b) und einen flexiblen Teil (1c) unterteilt ist, und im Inneren der Sonde (1) ein Optik- und Beleuchtungskanal (13a) ausgebildet ist, und zusätzlich in dem flexiblen Kabel (12b) Faserbündeln (12b4) und elektrische Drähte (12b3) geführt werden, die mit Anschlüssen zu peripheren Geräten abgeschlossen sind, **dadurch gekennzeichnet, dass** sich in der Kapsel (12a) eine Mikrokamera (12a6) mit einem System von Linsen (12a3) und Enden der Faseroptik (12b4) und elektrischen Kabeln (12b3) befindet, **dadurch gekennzeichnet, dass** der Durchmesser der Kapsel (12a) im Querschnitt größer ist als der Durchmesser des flexiblen Kabels (12b) und darüber hinaus, nach der Installation des Optik- und Beleuchtungsmoduls (12) im Endoskop, die Kapsel (12a) stabil in dem distalen Ende (1a) angeordnet ist, das durch beweglich verbundene Teile gebildet wird, **dadurch gekennzeichnet, dass** das distale Ende (1a) durch mindestens zwei Teile gebildet wird, die miteinander in einer trennbaren bzw. untrennbaren Weise verbunden sind, wobei die Teile, nach der Verbindung einen inneren Optik- und Beleuchtungskanal (1a) bilden, in dem die Kapsel (12a) des Optik- und Beleuchtungsmoduls (12) angeordnet wird und zusätzlich das distale Ende (1a) mit einer Klammer (1a2c) ausgerüstet ist, deren Länge an die Verengung zwischen der Kapsel (12a) und dem flexiblen Kabel (12b) des Optik- und Beleuchtungsmoduls (12) angepasst ist, um die Kapsel durch Abstützung ihrer Rückwand an einer Rückwärtsbewegung zu hindern.

2. Endoskop nach Anspruch 1, **dadurch gekennzeichnet, dass** das flexible Kabel (12b) des Optik- und Beleuchtungsmoduls (12) durch und im Inneren der Sonde (1), über ihre gesamte Länge, durch den Optik- und Beleuchtungskanal (13a) und durch mindestens einen Teil des Führungsanschlusses (2) geführt wird.

3. Endoskop nach Anspruch 1, **dadurch gekennzeichnet, dass** dass das flexible Kabel (12b) des Optik- und Beleuchtungsmoduls (12) im Inneren der Sonde (1) durch den Optik- und Beleuchtungskanal (13a) und durch den Führungsanschluss (2) und den Griff (3) geführt wird.

4. Endoskop nach einer der Ansprüche 1-3, **dadurch gekennzeichnet, dass** an der Außenfläche der Kapsel (12a) mindestens eine Führung (12a1), vorzugsweise drei Führungen, und über die gesamte Länge des Optik- und Beleuchtungskanals (13a) und an dessen Innenkontur mindestens eine Ausnehmung (13a1), vorzugsweise drei Ausnehmungen gebildet sind, die der Form der Führungen (12a1) der Kapsel (12a) entsprechen.

5. Endoskop nach einer der Ansprüche 1-4, **dadurch gekennzeichnet, dass** das flexible Kabel (12b) des Optik- und Beleuchtungsmoduls (12) mit einer Metallumhüllung und/oder einem Metallgeflecht bedeckt ist.

6. Endoskop nach einer der Ansprüche 1-5, **dadurch gekennzeichnet, dass** sich in der Kapsel (12a) des Optik- und Beleuchtungsmoduls (12) ein Temperaturschreiber (12a5) befindet.

7. Endoskop nach einer der Ansprüche 1-6 , **dadurch gekennzeichnet, dass** am distalen Ende (1a) ein Spiegel mit verstellbarem Winkel (1a4) angebracht ist.

8. Endoskop nach einer der Ansprüche 1-7, **dadurch gekennzeichnet, dass** das Bündel von Funktionskanälen (13) der Sonde (1) als Mehrkanalrohr ausgebildet ist und ein untrennbares Element bildet.

9. Endoskop nach Anspruch 2, **dadurch gekennzeichnet, dass** im Führungsanschluss (2) eine äußere Mündung des Optik- und Beleuchtungsmoduls (11) ausgebildet ist.

10. Endoskop nach Anspruch 3, **dadurch gekennzeichnet, dass** die Innenwände des Griffs (3) eine System von Funktionskanälen (3a,3b,3c,3d) und einen Optik- und Beleuchtungskanal (3e) bilden.

11. Endoskop nach Anspruch 8, **dadurch gekennzeichnet, dass** als Funktionskanäle (13) der Sonde (1) insbesondere der Luftkanal (13b), der Absaugkanal (13c), der Wasserkanal zur Linsenspülung (13d), der Wasserkanal zur Beobachtungsfeldspülung (13e), die Strangkanäle (13f) ausgeführt sind.

12. Endoskop nach Anspruch 11, **dadurch gekennzeichnet, dass** der Absaugkanal (3c) in dem Griff (3), der eine Verlängerung des Absaugkanals (13c), der Funktionskanäle (13), der Sonde (1) darstellt, mit einem Filter (3h) ausgestattet ist.

## Revendications

1. Endoscope souple équipé d'une poignée, qui est rallié à un tube d'insertion par un connecteur de guidage distal et le un tube d'insertion a la forme d'un élément tubulaire équipé d'un faisceau de canaux fonctionnels et terminé d'une pointe distale, **caractérisé en ce qu'**il est équipé d'un module optique et illuminant amovible et indépendant (12) contenant un tube élastique (12b), terminé d'une capsule (12a), et le tube d'insertion (1) est divisé en pointe distale (1a), section de flexion (1b) et partie flexible (1c) et à l'intérieur du tube d'insertion (1) un cannal optique et illuminant (13a) est formé et en plus, dans le tube élastique (12b) des faisceaux de fibres optiques (12b4) et des câbles électriques (12b3) sont fournis et terminés des connexions aux périphériques, **caractérisé en ce qu'**une micro caméra (12a6) équipée des lentilles (12a3) et des bornes du faisceau de fibres optiques (12b4) et des câbles électriques (12b3) est fournie dans la capsule (12a), **caractérisé en ce qu'**en coupe transversale, le diamètre de la capsule (12a) est plus grand du diamètre du tube élastique (12b) et en plus, si le module optique et illuminant (12) est installé dans dans l'endoscope, la capsule (12a) est fixée solidement dans la pointe distale (1a) qui est formée par des parties connectées de façon mobile, **caractérisé en ce que** la pointe distale (1a) est formée par au moins deux parties qui sont connectées séparablement ou inséparablement et les parties après la connexion forment un canal optique et illuminant interne (1a) dans lequel la capsule (12a) du module optique et illuminant (12) est installée et en plus la pointe distale (1a) est équipée d'un crochet (la2c) dont la longueur est adaptée au rétrécissement entre la capsule (12a) et le tube élastique (12b) du module optique et illuminant (12) de manière à empêcher la capsule de reculer en soutenant sa paroi arrière.

2. Endoscope selon la revendication 1, **caractérisé en ce que** le tube élastique (12b) du module optique et illuminant (12) est guidé à travers et à l'intérieur sur toute la longueur du tube d'insertion (1), à travers le cannal optique et illuminant (13a) et à travers au moins d'une partie du connecteur de guidage distal (2).

3. Endoscope selon la revendication 1, **caractérisé en ce que** le tube élastique (12b) du module optique et illuminant (12) est guidé à l'intérieur du tube d'insertion (1), à travers le cannal optique et illuminant (13a) et à travers le connecteur de guidage distal (2) et à travers la poignée (3).

4. Endoscope selon l'une des revendications 1 - 3, **caractérisé en ce que** sur la surface externe de la capsule (12a) au moins un guidage (12a1) est formé, de préférance trois guidges, et sur toute la longueur du cannal optique et illuminant (13a) et sur son contour interne au moins une cavité (13a1) est formée, de préférance trois cavités, dont la forme est adaptée aux guidages (12a1) de la capsule (12a).

5. Endoscope selon l'une des revendications 1 - 4, **caractérisé en ce que** le tube élastique (12b) du module optique et illuminant (12) est recouvert d'une gaine métallique et / ou d'une tresse métallique.

6. Endoscope selon l'une des revendications 1 - 5, **caractérisé en ce que** dans la capsule (12a) du module optique et illuminant (12) un enregistreur de température (12a5) est installé.

7. Endoscope selon l'une des revendications 1 - 6, **caractérisé en ce qu'**un miroir à angle variable (1a4) est attaché à la pointe distale (1a).

8. Endoscope selon l'une des revendications 1 - 7, **caractérisé en ce que** le faisceau de canaux fonctionnels (13) du tube d'insertion (1) est formé en tant que tube multi-cannaux constituant un élément solide.

9. Endoscope selon la revendication 2, **caractérisé en ce que** dans le connecteur de guidage distal (2) une entrée externe du module optique et illuminant (11) est réalisée.

10. Endoscope selon la revendication 3, **caractérisé en ce que** le faisceau de canaux fonctionnels (3a, 3b, 3c, 3d) et le cannal optique et illuminant (3e) sont formés par les parois internes de la poignée (3).

11. Endoscope selon la revendication 8, **caractérisé en ce que** les canaux fonctionnels (13) du tube d'insertion (1), sont notamment un cannal d'air (13b), un cannal de succion (13c), un canal d'eau de rinçage des lentilles (13d), un canal d'eau de rinçage de la zone d'observation (13e), un cannal de câbles d'angulation (13f).

12. Endoscope selon la revendication 11, **caractérisé en ce que** le cannal de succion (3c), formé dans la poignée (3), constituant un prolongement du canal de succion (13c) des cannaux fonctionnels (13) du tube d'insertion (1) est équipé d'un filtre (3h).
